(19) Europäisches Patentamt
European Patent Office
Office européen des brevets

(11) **EP 4 371 967 A1**

(12) **EUROPEAN PATENT APPLICATION**

(43) Date of publication:
**22.05.2024 Bulletin 2024/21**

(21) Application number: **23206601.9**

(22) Date of filing: **30.10.2023**

(51) International Patent Classification (IPC):
*C04B 41/00* [(2006.01)]    *C04B 41/52* [(2006.01)]
*C04B 35/486* [(2006.01)]    *C04B 35/632* [(2006.01)]
*C04B 35/634* [(2006.01)]    *C04B 35/638* [(2006.01)]
*A61C 13/00* [(2006.01)]    *A61C 13/083* [(2006.01)]
*A61K 6/818* [(2020.01)]    *A61K 6/822* [(2020.01)]
*C04B 111/00* [(2006.01)]    *C04B 111/82* [(2006.01)]

(52) Cooperative Patent Classification (CPC):
(C-Sets available)
**C04B 41/009; A61C 13/0022; A61C 13/083;
A61K 6/818; C04B 35/486; C04B 35/632;
C04B 35/63488; C04B 35/638; C04B 41/52;**
C04B 2111/00405; C04B 2111/00836;
C04B 2111/82; C04B 2235/3224; C04B 2235/3225;
C04B 2235/3272;         (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.10.2022 JP 2022173775**

(71) Applicant: **Shofu Inc.
Kyoto-shi, Kyoto 605-0983 (JP)**

(72) Inventors:
• **NONAKA, Kazumichi
Kyoto, 605-0983 (JP)**
• **TAKAHASHI, Shuhei
Kyoto, 605-0983 (JP)**

(74) Representative: **Eisenführ Speiser
Patentanwälte Rechtsanwälte PartGmbB
Gollierstraße 4
80339 München (DE)**

(54) **ZIRCONIA MILL BLANK**

(57)     To provide a zirconia mill blank for dental cutting and machining that can impart translucency gradation and color gradation similar to a natural tooth to a zirconia perfect sintered body without the need for special equipment.

To provide a zirconia mill blank for dental cutting and machining comprising
two opposing surfaces and a side surface between the two opposing surfaces, wherein
the side surface has a rectangular shape in a side view of the zirconia mill blank for dental cutting and machining, and
in a case in which
one of the two opposing surfaces is defined as surface A,
the other surface of the two opposing surfaces is defined as surface B,
a surface which is parallel to the surface A and is located at a position of 1.0 mm to 1.5 mm from the surface A toward the surface B in a direction parallel to the side surface is defined as surface C,
points which are set to having 0.5 mm of distance on the straight line in the direction parallel to the side surface from the surface C toward the surface B in the area from the surface C to the surface B are defined as point P(1), point P(2), to point P(n) in the order from the surface C, a stabilizer concentration (mol%) at each point is defined as SP(1), SP(2), to SP(n) (n ≥ ((shortest distance (mm) between the surface C and the surface B) - 2)/0.5),
the maximum value of the stabilizer concentration (mol%) is defined as SPmax,
the minimum value of the stabilizer concentration (mol%) is defined as SPmin, and
the maximum value of ΔSP(m) defined by the following formula is defined as ΔSPmax,

the zirconia mill blank for dental cutting and machining has a portion where the value of "SPmax - Spmin" which is a difference between the SPmax and the SPmin is 0.3 or more, and the ΔSPmax is less than 0.5.

$$\Delta SP(m) = |SP(m) - SP(m+1)| \ (m=1, 2, \ldots n-1)$$

**(Cont. next page)**

(52) Cooperative Patent Classification (CPC): (Cont.)
C04B 2235/3279; C04B 2235/443;
C04B 2235/449; C04B 2235/616;
C04B 2235/6567; C04B 2235/75; C04B 2235/77;
C04B 2235/775; C04B 2235/9615;
C04B 2235/9653

C-Sets
**C04B 41/009, C04B 35/48, C04B 38/00;**
**C04B 41/52, C04B 41/4539, C04B 2103/0014,**
**C04B 41/5009, C04B 41/5042;**
**C04B 41/52, C04B 41/4539, C04B 2103/0021,**
**C04B 41/5009**

**Description**

BACKGROUND OF THE INVENTION

Field of the Invention

The present invention relates to a zirconia mill blank for dental cutting and machining.

Description of the Related Art

In recent years, techniques to prepare a prosthesis device by the cutting and machining which uses the dental CAD/CAM system has been spread rapidly and it has been becoming possible to easily prepare prosthesis devices by cutting and machining the blanks which are made of ceramic materials such as zirconia, alumina and lithium disilicate glass, and resin materials such as an acrylic resin and a hybrid resin.

In particular, the zirconia has been clinically applied in various cases because of its high strength. On the other hand, the perfect sintered zirconia (hereinafter, also referred to as "zirconia perfect sintered body") has very high hardness and therefore cannot be cut and machined using a dental CAD / CAM system. Thus, a zirconia which is not fully sintered but is calcined at a low firing temperature to adjust to a hardness that enables to cut has been used as a zirconia mill blank for dental cutting and machining.

A general zirconia mill blank for dental cutting and machining is prepared by molding a zirconia powder by press molding or the like and then calcining at 800 to 1200 °C.

The properties of the zirconia perfect sintered body (fully sintered body) are influenced by the properties of the used zirconia powder.

International Publication No. 2021/023791 discloses a zirconia mill blank for dental cutting and machining that comprises multiple layers having a different stabilizer content and a different colorant content. The zirconia perfect sintered body prepared from the zirconia mill blank has translucency gradation and color gradation.

However, for example, a boundary between layers is visible in this sintered body, the sintered body does not have translucency gradation and color gradation similar to a natural tooth, and therefore it has been difficult to apply to the case where high aesthetic property is required such as a front tooth portion.

SUMMARY OF THE INVENTION

Technical Problem

An object of the present invention is to provide a zirconia mill blank for dental cutting and machining that can impart translucency gradation and color gradation similar to a natural tooth to a zirconia perfect sintered body without the need for special equipment.

Solution to Problem

The present inventors made a study of a zirconia mill blank for dental cutting and machining that can impart translucency gradation and color gradation similar to a natural tooth to a zirconia perfect sintered body without the need for special equipment. As a result, the present inventors have found that it is particularly important that the stabilizer concentration at each portion in the zirconia mill blank for dental cutting and machining satisfies a certain relationship for imparting translucency gradation and color gradation similar to a natural tooth to a zirconia perfect sintered body. The details of the present invention are described below.

The zirconia mill blank for dental cutting and machining of the present invention comprises

two opposing surfaces and a side surface between the two opposing surfaces, wherein
the side surface has a rectangular shape in a side view of the zirconia mill blank for dental cutting and machining, and
in a case in which

one of the two opposing surfaces is defined as surface A,
the other surface of the two opposing surfaces is defined as surface B,
a surface which is parallel to the surface A and is located at a position of 1.0 mm to 1.5 mm from the surface A toward the surface B in a direction parallel to the side surface is defined as surface C,
points which are set to having 0.5 mm of distance on the straight line in the direction parallel to the side surface from the surface C toward the surface B in the area from the surface C to the surface B are defined as point P(1), point P(2), to point P(n) in the order from the surface C,
a stabilizer concentration (mol%) at each point is defined as SP(1), SP(2), to SP(n) (n $\geq$ ((shortest distance (mm) between the surface C and the surface B) - 2)/0.5),
the maximum value of the stabilizer concentration (mol%) is defined as SPmax,
the minimum value of the stabilizer concentration (mol%) is defined as SPmin, and
the maximum value of $\Delta$SP(m) defined by the following formula is defined as $\Delta$SPmax,

the zirconia mill blank for dental cutting and machining has a portion where the value of "SPmax - Spmin" which is a difference between the SPmax and the SPmin is 0.3 or more, and the $\Delta$SPmax is less than 0.5.

$$\Delta SP(m) = |SP(m) - SP(m+1)| \ (m = 1, 2, \ldots n\text{-}1)$$

In the present invention, the $\Delta$SPmax may be less than 0.3.

In the present invention, the $\Delta$SPmax may be less than 0.10.

In the present invention, the number of three consecutive points that satisfy the following formula may be less than 3.

$$\Delta\Delta SP(m) = |\Delta SP(m) - \Delta SP(m+1)| > 0.1 \ (m = 1, 2, \ldots n\text{-}1)$$

In the present invention, the SPmax which is the maximum value of stabilizer concentration may be 6.0 mol% or more.

In the present invention, in a case in which a contrast ratio is measured for a sintered test specimen having a thickness of 1.0 mm which is prepared by sintering a test specimen having a thickness of 2.0 mm cut out from a region from the surface C to the surface B at 1550°C for 1 hour to prepare a sinterd body and adjusting a thickness of the sinterd body to 1.0mm, the lowest contrast ratio in the region may be 0.70 or less.

In the present invention, the zirconia mill blank for dental cutting and machining may be colored.

In the present invention, the zirconia mill blank for dental cutting and machining may not have stacked structure.

In the present invention, a stabilizer compound and/or a colorant compound may be supported in a pore.

In the present invention, the point P(1) on the surace C may be positioned at a intersection of the surface C and the side surface.

In the present invention, the point P(1) on the surace C may be positioned at a position closest proximity to the surface B on the surface C.

In the present invention, in the entire of the surface C, the value of "SPmax - Spmin" may be 0.3 or more and the $\Delta$SPmax is less than 0.5.

$$\Delta SP(m) = |SP(m) - SP(m+1)| \ (m = 1, 2, \ldots n\text{-}1)$$

The present invention also provides a method for imparting translucency gradation and color tone gradation to a zirconia mill blank for dental cutting and machining, comprising
a step of impregnating the zirconia mill blank for dental cutting and machining with at least two types of impregnating solutions.

Advantageous Effects of Invention

The zirconia mill blank for dental cutting and machining of the present invention can impart translucency gradation and color gradation similar to a natural tooth to a zirconia perfect sintered body.

DETAILED DESCRIPTION OF THE PREFERRED EMBODIMENTS

Hereinafter, constituent elements in the present invention are described in detail. The present invention relates to a zirconia mill blank for dental cutting and machining which has a feature in that the stabilizer concentration at each portion satisfies a certain relationship. Specifically, the zirconia mill blank for dental cutting and machining of the present invention comprises

two opposing surfaces and a side surface between the two opposing surfaces, wherein

the side surface has a rectangular shape in a side view of the zirconia mill blank for dental cutting and machining, and in a case in which

one of the two opposing surfaces is defined as surface A,
the other surface of the two opposing surfaces is defined as surface B,
a surface which is parallel to the surface A and is located at a position of 1.0 mm to 1.5 mm from the surface A toward the surface B in a direction parallel to the side surface is defined as surface C,
points which are set to having 0.5 mm of distance on the straight line in the direction parallel to the side surface from the surface C toward the surface B in the area from the surface C to the surface B are defined as point P(1), point P(2), to point P(n) in the order from the surface C,
a stabilizer concentration (mol%) at each point is defined as SP(1), SP(2), to SP(n) (n ≥ ((shortest distance (mm) between the surface C and the surface B) - 2)/0.5),
the maximum value of the stabilizer concentration (mol%) is defined as SPmax,
the minimum value of the stabilizer concentration (mol%) is defined as SPmin, and
the maximum value of ΔSP(m) defined by the following formula is defined as ΔSPmax,

the zirconia mill blank for dental cutting and machining has a portion where the value of "SPmax - Spmin" which is a difference between the SPmax and the SPmin is 0.3 or more, and the ΔSPmax is less than 0.5.

$$\Delta SP(m) = |SP(m) - SP(m+1)| \ (m=1, 2, \ldots n-1)$$

In the present invention, the side view of the zirconia mill blank for dental cutting and machining means the zirconia mill blank for dental cutting and machining in a state of facing forward to the side surface of the zirconia mill blank for dental cutting and machining. In the present invention, the surface C may be a virtual surface.

In the present invention, the value of "SPmax - Spmin" which is a difference between the SPmax and the SPmin which are the maximum value and the minimum value of the stabilizer concentration (mol%) may be 0.5 or more, may be 0.6 or more, may be 0.7 or more and may be 0.8 or more. When the value of "SPmax - Spmin" is 0.5 or more, it is possible to obtain translucency gradation similar to a natural tooth. When the value of "SPmax - Spmin" is less than 0.3, it is not possible to obtain translucency gradation similar to a natural tooth.

The ΔSPmax which is the maximum value of ΔSP(m) may be less than 0.4, may be less than 0.3, and may be less than 0.1. When the ΔSPmax is 0.5 or more, the change in translucency becomes rapid, and it is not possible to obtain natural color tone gradation, for example, a boundary is visible between two points. When the ΔSPmax is less than 0.3, the change in translucency becomes gradual and it is possible to obtain more natural color tone gradation. When the ΔSPmax is less than 0.1, the change in translucency becomes even more gradual, therefore, it is possible to obtain natural gradation in which there is substantially no boundary between two points.

In the present invention, the number of three consecutive points that satisfy the following formula may be less than 3. When the the number of three consecutive points that satisfy the following formula is 3 or more, there is a tendency that it is not possible to obtain natural gradation.

$$\Delta\Delta SP(m) = |\Delta SP(m) - \Delta SP(m+1)| > 0.1 \ (m = 1, 2, \ldots n-1)$$

In the present invention, the SPmax which is the maximum value of the stabilizer concentration may be 6.0 mol% or more. Further, in the present invention, the SPmax which is the maximum value of the stabilizer concentration may be 7.0 mol% or less. When the SPmax which is the maximum value of the stabilizer concentration is less than 6.0 mol%, there is a tendency that it is not possible to obtain translucency similar to a natural tooth. When the SPmax which is the maximum value of the stabilizer concentration exceeds 7.0 mol%, there is a tendency that translucency decreases and it is not possible to obtain translucency similar to a natural tooth.

In the present invention, in a case in which a contrast ratio is measured for a sintered test specimen having a thickness of 1.0 mm which is prepared by sintering a test specimen having a thickness of 2.0 mm cut out from a region from the surface C to the surface B at 1550°C for 1 hour to prepare a sinterd body and adjusting a thickness of the sinterd body to 1.0mm, the lowest contrast ratio in the region may be 0.70 or less, and may be 0.65 or less. When the contrast ratio exceeds 0.70, there is a case in which it is not possible to obtain translucency similar to a natural tooth.

The zirconia mill blank for dental cutting and machining of the present invention may be not colored. In the case in which the zirconia mill blank for dental cutting and machining is not colored, there is no need to mix a plurarity of powders in preparing a disc.

The zirconia mill blank for dental cutting and machining of the present invention may not have stacked structure.

Since the zirconia mill blank for dental cutting and machining does not have a stacked structure, it is possible to obtain natural gradation without a layer interface.

The surface A and the surface B, which constitute the two opposing surfaces, and the surface C which is parallel to the surface A may be plane surfaces and may be other surfaces other than a plane surface, such as a curved surface or a surface with unevenness.

In the present invention, points which are set to having 0.5 mm of distance on the straight line perpendicular to the surface C in the area from the surface C to the surface B are defined as point $P(1)$, point $P(2)$, to point $P(n)$ in the order from the surface C. Further, the stabilizer concentration (mol%) at each point is defined as $SP(1)$, $SP(2)$, to $SP(n)$. The "n" is an integer satisfying "n ≥ ((shortest distance (mm) between the surface C and the surface B) - 2)/0.5".

In the zirconia mill blank for dental cutting and machining of the present invention, the position of the point $P(1)$ on the surface C may be arbitrarily determined. In the present invention, the zirconia mill blank for dental cutting and machining is required to have a portion where the value of "SPmax - Spmin" which is a difference between the SPmax and the SPmin which are the maximum value and the minimum value of the stabilizer concentration (mol%) is 0.3 or more and the ΔSPmax which is the maximum value of ΔSP(m) is less than 0.5, but may have a portion where the value of "SPmax - Spmin" which is a difference between the SPmax and the SPmin is less than 0.3 and/or the ΔSPmax is 0.5 or more.

The the point $P(1)$ on the surface C may be, for example, positioned at a intersection of the surface C and the side surface. The point $P(1)$ on the surface C may be, for example, positioned at a position closest proximity to the surface B on the surface C.

In the present invention, in the entire of the surface C, the value of "SPmax - Spmin" may be 0.3 or more and the ΔSPmax may be less than 0.5.

The stabilizer contained in the zirconia mill blank for dental cutting and machining of the present invention is not particularly limited, but yttria may be used because it is easily available and provides excellent properties. For other stabilizers, erbium oxide, neodymium oxide, praseodymium oxide and terbium oxide which are also effective as a coloring agent may be used.

The zirconia mill blank for dental cutting and machining of the present invention may contain a stabilizer compound and/or a colorant compound which are not solid solved. Specifically, it is preferable that the stabilizer compound and/or the colorant compound used in the present invention is a water-soluble compound consisting of any one of a oxide, a halogen compound, a nitrate, a sulfate and an organic acid salt. Specific examples of water-soluble stabilizer compound and/or water-soluble colorant compound include yttrium chloride, yttrium nitrate, yttrium acetate, yttrium carboxylate, yttrium sulfate, yttrium carbonate, erbium chloride, erbium nitrate, erbium acetate, erbium carboxylate, erbium sulfate, erbium carbonate, neodymium chloride, neodymium nitrate, neodymium acetate, neodymium carboxylate, neodymium sulfate, neodymium carbonate, praseodymium chloride, praseodymium nitrate, praseodymium acetate, praseodymium carboxylate, praseodymium sulfate, praseodymium carbonate, terbium chloride, terbium nitrate, terbium acetate, terbium carboxylate, terbium sulfate, terbium carbonate, iron chloride, iron nitrate, iron acetate, nickel chloride, nickel nitrate, nickel acetate and the like.

Among a water-soluble stabilizer compound and/or a water-soluble colorant compound, an organic acid salt is particularly preferable from the viewpoint of low decomposition temperature and low contamination of the firing furnace and the like. Specific examples include yttrium acetate and yttrium carboxylate. Organic acid salts decompose at lower temperatures than inorganic salts such as halogen compounds, nitrates, and sulfates. In the case that the decomposition temperature is high, there is a case where it is difficult to impart sufficient translucency and strength to a zirconia perfect sintered body because pore remains in the sintering process.

In the present invention, a stabilizer compound and/or a colorant compound may be supported in a pore. By supporting a stabilizer compound and/or a colorant compound in a pore, it is possible to improve translucency and to impart desired color tone.

The zirconia mill blank for dental cutting and machining in the present invention may contain additives other than a stabilizer and a colorant. Specific examples of additives include compounds of aluminum, gallium, lanthanum, niobium, and tantalum. By addition of these additives, it is possible to control sintering behavior, to reduce translucency, and to improve fracture toughness. These additives may or may not be solid-solved in zirconia.

As the zirconia powder used for preparing the zirconia mill blank for dental cutting and machining of the present invention, any known zirconia powder may be used without any limitations. Specifically, it is preferable that zirconia powder used for preparing the zirconia mill blank for dental cutting and machining of the present disclosure is prepared by a hydrolysis method. More specifically, the zirconia powder is prepared by a method which comprises heating a solution in which a zirconium salt and a yttrium compound are mixed and dissolved to cause a hydrolysis reaction, drying and firing the generated sol to prepare a zirconia powder, and pulverizing and granulating the powder. Further, by mixing an aluminum compound before this pulverization step, an alumina-containing zirconia powder is prepared.

The primary particle diameter of a zirconia powder used for preparing the zirconia mill blank for dental cutting and machining in the present invention may be within a range of 1 to 500 nm. When the primary particle diameter is less than 1 nm, there is a tendency that it is difficult to impart sufficient strength, although translucency of the zirconia sintered body is improved. On the other hand, when the primary particle diameter is more than 500 nm, there is a tendency that it is difficult to impart sufficient strength to the zirconia sintered body.

The zirconia mill blank for dental cutting and machining of the present invention may contain a coloring material. Specific examples thereof include iron oxide for imparting yellow color and erbium for imparting red color. In addition, there is no problem at all even if a coloring material containing an element such as cobalt, manganese and chromium for adjusting color in addition to these coloring materials is used together. In the present invention, the tooth color can be easily imparted by including the coloring material.

The relative density of the zirconia sintered body prepared by firing the zirconia mill blank for dental cutting and machining of the present invention at 1550 °C may be 98% or more of the theoretical density. The relative density is determined by the measured density/the theoretical density. When the relative density is less than 98%, there is a tendency that strength and translucency are lowered.

A crystal phase of the zirconia mill blank for dental cutting and machining of the present invention may be tetragonal and/ or cubic. When the crystal phase is monoclinic phase, there is a case in which sufficient translucency is not imparted after perfect sintering of zirconia.

A preparing method of the zirconia mill blank for dental cutting and machining of the present invention is not particularly limited, and any known preparing methods can be used without any problem. Specifically, it is preferable to be prepared by molding a zirconia powder by press molding. Furthermore, it may be prepared by a multilayer molding in which zirconia powders having different compositions such as a colorant concentration and a stabilizer concentration are press-molded in multiple stages. The zirconia mill blank for dental cutting and machining in the present invention may not have stacked structure.

The zirconia mill blank for dental cutting and machining of the present invention may be subjected to isostatic pressing by cold isostatic pressing (CIP treatment) after the press molding.

The maximum load pressure of CIP treatment in the present invention may be 50 Mpa or more. When the maximum load pressure is less than 50 MPa, there is a case where sufficient translucency and strength is not imparted to the zirconia sintered body.

The holding time at the maximum load pressure of the CIP treatment in the present invention is not particularly limited, but in general, may be within a range of 0 to 150 seconds and may be within a range of 0 to 60 seconds.

The time period required for the series of processes from the start of pressurization to the end of depressurization in the CIP treatment is not particularly limited, but may be usually within a range of 30 seconds to 10 minutes and may be within a range of 3 minutes to 7 minutes. When the time is too short, a molding body may be destroyed, and when the time is too long, production efficiency worsens, and therefore these are not preferable.

A calcination temperature of the zirconia mill blank for dental cutting and machining of the present invention is preferably within a range of 800 to 1200 °C. When the calcination temperature is less than 800 °C, because Vickers hardness and/ or bending strength become too low and therefore there is a tendency that chipping and breakage easily occur in the cutting and machining. On the other hand, when the calcination temperature is more than 1200 °C, because Vickers hardness and/ or bending strength become too high and therefore there is a tendency that a milling bar of a milling machine is heavily consumed to raise a running cost.

The process of preparing a zirconia mill blank for dental cutting and machining in the present invention may include a step of permeating with an impregnating solution containing a metal ion. Specifically, at least one type of impregnating solution is permeated into the zirconia calcined body, and the metal compound is supported in a zirconia pore by drying, degreasing, and other processes. Depending on the type of a metal ion, this metal compound functions as a stabilizer, a colorant, or a sintering behavior adjusting agent. By this step, it is possible to impart the desired translucency, color tone, mechanical property and the like to desired areas of the zirconia mill blank for dental cutting and machining. It is possible to use two or more kinds of impregnating solutions. In this case, smooth translucency gradation, color gradation and/or mechanical property gradation are imparted to the zirconia mill blank by ion diffusion between the impregnating solutions. An impregnating solution that does not contain metal ions may also be used for ion diffusion.

Examples of a metal ion that functions as a stabilizer include yttrium ion, erbium ion, ytterbium ion, magnesium ion, praseodymium ion and neodymium ion.

Examples of a metal ion that functions as a colorant include iron ion, nickel ion and cobalt ion.

Examples of a metal ion that functions as a sintering behavior adjusting agent include aluminum ion, gallium ion and lanthanum ion.

At least one of the metal ion contained in the impregnating solution may be a rare earth metal ion. Specific examples of the rare earth metal ion include an yttrium ion. By containing a rare earth metal ion, it is possible to improve the translucency of the zirconia sintered body and to color the zirconia sintered body. The metal ion may be only a rare earth metal ion.

At least one of the metal ion contained in the impregnating solution may be a transition metal ion. Specific transition metal ion is an iron ion. By containing a transition metal ion, it is possible to color a zirconia sinterd body. The metal ion may be only a transition metal ion.

At least one of the metal ion contained in the impregnating solution may be any of aluminum ion, gallium ion and indium ion. By containing any of aluminum ion, gallium ion and indium ion, it is possible to improve the sinterability of the zirconia sintered body and to improve the translucency of the zirconia sintered body. The metal ion may be only aluminum ion, gallium ion and/or indium ion. The metal ion may be only a rare earth metal ion, a transition metal ion, aluminum ion, gallium ion and/or indium ion.

The metal ion concentration of the impregnating solution is not particularly limited, but in the case of an yttrium ion, for example, it may be about within a range of 2.0 mass% to 10.0 mass%. When it is 2.0 mass% or less, there is a case that the amount of supported yttrium is small and sufficient characteristic is not obtained. When it is 10.0 mass% or more, there is a case that the amount of supported yttrium is large and physical property is adversely affected. Since the optimum amount of supported metal changes depending on the type of metal and the desired characteristics, the metal ion concentration of the impregnating solution is preferably determined from the desired amount of supported metal and the solubility of a metal ion source in the solvent.

The solvent used in the impregnating solution is not particularly limited, but water is preferable since it is easily available and is easy to handle.

The impregnating solution may be added with acid, base, or pH adjusting agent for the purpose of controlling the pH. The acid, base or pH adjusting agent to be used is not particularly limited, but it is possible to use an organic acid such as acetic acid, citric acid and the like or aqueous ammonia from the viewpoint of leaving no residue on the zirconia mill blank for dental cutting and machining.

The impregnating solution may contain a precipitant. A precipitant precipitates a metal compound by heat treatment or other operation after permeation of the impregnating solution, and specific examples include urea and hexamethylenetetetramine. Urea may be used since it is easily available and is easy to handle. By this, segregation of a metal compound can be suppressed.

The impregnating solution may contain various additives. Examples of additives include glycols and polymers that adjust the viscosity of the impregnating solution, and chelating agents that increase the stability of a metal ion. Specific Examples of the former include ethylene glycol, propylene glycol, polyethylene glycol and the like. Specific Examples of the latter include citric acid, ammonium ethylenediaminetetraacetate and the like.

As the impregnation method of impregnating the zirconia calcined body body with the impregnating solution, any method can be used without particular limitation as long as the impregnating solution can infiltrate into the pores of the zirconia mill blank for dental cutting and machining. In a simple and preferable method, the whole and/ or a part of the zirconia mill blank for dental cutting and machining is immersed in the impregnating solution. In this case, the impregnating solution can infiltrate by the capillarity into an inside of the porous zirconia molded body. The immersion time is 3 minutes or more, preferably 10 minutes to 10 hours.

The impregnating solution may be permiated in the whole of the zirconia mill blank for dental cutting and machining and may be permiated in an arbitrary portion of the zirconia mill blank for dental cutting and machining. The method of permiating only an arbitrary portion is not particularly limited, and examples thereof include control of the weight and volume of the impregnating solution and control of the impregnation time.

A plurality of impregnating solutions may be used for one zirconia mill blank for dental cutting and machining. In a specific example, after permiating the first type impregnating solution from one end, the second type impregnating solution is impregnated from the same end or the other end.

The atmosphere in impregnating the impregnating solution is not particularly limited, and an ambient air, an inert gas and the like can be used under normal pressure, reduced pressure, and pressurized conditions. Since no special facilities is required, it is preferable to perform under normal pressure in an ambient air.

It is possible to comprise a drying step after the permiating step. The drying step is a step of drying the the zirconia mill blank for dental cutting and machining in which a metal compound is deposited. The drying temperature may be within a range of 50 to 200 °C, and the drying time may be within a range of 15 minutes to 20 hours. It is possible to sufficiently vaporize the solvent after drying. In some cases, the heat treatment step may be carried out following this drying step. The drying step may also be included in the heat treatment step.

Since a solvent, unreacted product, by-product and the like remain in the zirconia mill blank for dental cutting and machining in which a metal compound is deposited and supported, prepared in such a way, it is possible to perform a heat treatment. The heat treatment method is not particularly limited, but heat treatment under normal atmospheric pressure may be used since no special facilities is required. The heat treatment temperature is not particularly limited, but may be within a range of 500 °C to 1200 °C. By heat treatment, impurities, organic substances and odors can be removed.

By such preparing method, a zirconia mill blank for dental cutting and machining of the present invention can be prepared. The prepared zirconia mill blank for dental cutting and machining is severed, cut, and surface polished so as to have a desired size as necessary.

The method for perfect sintering the zirconia mill blank for dental cutting and machining of the present invention is not particularly limited, but a simple and preferred method is to firing at normal pressure. The firing temperature is not limited, but may be within a range from 1450 to 1600 °C, and may be within a range from 1500 to 1600 °C. The holding time at the firing temperature is not particularly limited, but may be within a range of 1 minute to 12 hours, and may be within a range of 2 to 4 hours. The temperature increase rate is not particularly limited, but may be within a range from 1 to 400°C/min and from 3 to 100°C/h.

The type of a prosthesis device prepared by cutting and machining the zirconia mill blank for dental cutting and machining according to the present invention is not limited particularly, and there is no problem at all even if the prosthesis device is any of an inlay, an onlay, a veneer, a crown, a bridge and the like. Therefore, a shape of a zirconia mill blank for dental cutting and machining which is cut and machined for preparing a prosthesis device is not limited particularly, and any zirconia mill blank for dental cutting and machining can be used even if the zirconia mill blank for dental cutting and machining has any shape such as a block shape corresponding to an inlay, an onlay, a veneer, a crown and the like and a disk shape corresponding to a bridge.

[Examples]

Hereinafter, the present invention is described by way of Examples in more detail, and specifically, but the present invention is not limited to these Examples.

[Preparation of zirconia calcined body 1]

Zirconia powder containing 5.5 mol% of solid-solved yttrium in an amount of 486g was filled in a mold (φ100 mm), and press molding (surface pressure: 50 MPa) was performed to obtain a molded body. Further, the molded body was subjected to CIP treatment (maximum load pressure: 200 MPa, holding period: 1 minute). Thereafter, calcination was performed in an electric furnace (1000 °C, 30 minutes) to prepare a zirconia calcined body having a diameter of 98.5 mm and a thickness of 18 mm.

[Preparation of zirconia calcined body 2]

A zirconia calcined body was prepared in the same manner as the zirconia calcined body 1 expect that zirconia powder containing 4.0 mol% of solid solved yttrium was used.

[Preparation of zirconia calcined body 3]

A zirconia calcined body was prepared in the same manner as the zirconia calcined body 1 expect that zirconia powder containing 3.0 mol% of solid solved yttrium was used.

[Preparation of zirconia calcined body 4]

Zirconia powder containing 4.0 mol% of solid-solved yttrium in an amount of 170g was filled in a mold (φ100 mm), and the upper surface was scraped and flattened. Then, zirconia powder containing 4.4 mol% of solid-solved yttrium in an amount of 73g was filled, and the upper surface was scraped and flattened. Then, zirconia powder containing 4.8 mol% of solid-solved yttrium in an amount of 50g was filled, and the upper surface was scraped and flattened. Then, zirconia powder containing 5.1 mol% of solid-solved yttrium in an amount of 50g was filled, and the upper surface was scraped and flattened. Then, zirconia powder containing 5.5 mol% of solid-solved yttrium in an amount of 143g was filled, and the upper surface was scraped and flattened. Then, press molding (surface pressure: 50 MPa) was performed to obtain a molded body. Further, the molded body was subjected to CIP treatment (maximum load pressure: 200 MPa, load pressure after releasing: 0 MPa, holding time: 1 minute). Thereafter, calcination was performed in an electric furnace (1000 °C, 30 minutes) to prepare a zirconia calcined body.

[Preparation of zirconia calcined body 5]

Zirconia powder containing 3.0 mol% of solid-solved yttrium in an amount of 170g was filled in a mold (φ100 mm), and the upper surface was scraped and flattened. Then, zirconia powder containing 3.6 mol% of solid-solved yttrium in an amount of 73g was filled, and the upper surface was scraped and flattened. Then, zirconia powder containing 4.3 mol% of solid-solved yttrium in an amount of 50g was filled, and the upper surface was scraped and flattened. Then, zirconia powder containing 4.9 mol% of solid-solved yttrium in an amount of 50g was filled, and the upper surface was scraped and flattened. Then, zirconia powder containing 5.5 mol% of solid-solved yttrium in an amount of 143g was filled, and the upper surface was scraped and flattened. Then, press molding (surface pressure: 50 MPa) was performed to obtain a molded body. Further, the molded body was subjected to CIP treatment (maximum load pressure: 200 MPa, load pressure after releasing: 0 MPa, holding time: 1 minute). Thereafter, calcination was performed in an electric furnace (1000 °C, 30 minutes) to prepare a zirconia calcined body.

[Preparation of zirconia calcined body 6]

Zirconia powder containing 4.6 mol% of solid-solved yttrium in an amount of 170g was filled in a mold (φ100 mm), and the upper surface was scraped and flattened. Then, zirconia powder containing 4.8 mol% of solid-solved yttrium in an amount of 73g was filled, and the upper surface was scraped and flattened. Then, zirconia powder containing 5.1 mol% of solid-solved yttrium in an amount of 50g was filled, and the upper surface was scraped and flattened. Then, zirconia powder containing 5.3 mol% of solid-solved yttrium in an amount of 50g was filled, and the upper surface was scraped and flattened. Then, zirconia powder containing 5.5 mol% of solid-solved yttrium in an amount of 143g was filled, and the upper surface was scraped and flattened. Then, press molding (surface pressure: 50 MPa) was performed to obtain a molded body. Further, the molded body was subjected to CIP treatment (maximum load pressure: 200 MPa, load pressure after releasing: 0 MPa, holding time: 1 minute). Thereafter, calcination was performed in an electric furnace (1000 °C, 30 minutes) to prepare a zirconia calcined body.

[Preparation of zirconia calcined body 7]

Zirconia powder containing 3.0 mol% of solid-solved yttrium in an amount of 170g was filled in a mold (φ100 mm), and the upper surface was scraped and flattened. Then, zirconia powder containing 3.3 mol% of solid-solved yttrium in an amount of 32g was filled, and the upper surface was scraped and flattened. Then, zirconia powder containing 3.6 mol% of solid-solved yttrium in an amount of 32g was filled, and the upper surface was scraped and flattened. Then, zirconia powder containing 3.9 mol% of solid-solved yttrium in an amount of 32g was filled, and the upper surface was scraped and flattened. Then, zirconia powder containing 4.2 mol% of solid-solved yttrium in an amount of 32g was filled, and the upper surface was scraped and flattened. Then, zirconia powder containing 4.5 mol% of solid-solved yttrium in an amount of 32g was filled, and the upper surface was scraped and flattened. Then, zirconia powder containing 4.8 mol% of solid-solved yttrium in an amount of 32g was filled, and the upper surface was scraped and flattened. Then, zirconia powder containing 5.1 mol% of solid-solved yttrium in an amount of 32g was filled, and the upper surface was scraped and flattened. Then, zirconia powder containing 5.4 mol% of solid-solved yttrium in an amount of 32g was filled, and the upper surface was scraped and flattened. Then, zirconia powder containing 5.7 mol% of solid-solved yttrium in an amount of 32g was filled, and the upper surface was scraped and flattened. Then, zirconia powder containing 6.0 mol% of solid-solved yttrium in an amount of 32g was filled, and the upper surface was scraped and flattened. Then, press molding (surface pressure: 50 MPa) was performed to obtain a molded body. Further, the molded body was subjected to CIP treatment (maximum load pressure: 200 MPa, load pressure after releasing: 0 MPa, holding time: 1 minute). Thereafter, calcination was performed in an electric furnace (1000 °C, 30 minutes) to prepare a zirconia calcined body.

(Preparation of impregnating solution)

Table 1 shows the compositions of the impregnating solutions. According to the compositions shown in Table 1, the solution 1 to the solution 10 in amount of 100 g were prepared by adding a metal salt and an additive to ion-exchanged water and stirred for 1 hour, and were used as impregnating solutions.

[Table 1]

|  | Solution 1 | Solution 2 | Solution 3 | Solution 4 | Solution 5 |
|---|---|---|---|---|---|
| Solvent | Ion exchange water | Ion exchange water | Ion exchange water | Ion exchange water | Ion exchange water |
| Metal salt 1 | Yttrium nitrate n-hydrate | Iron(III) nitrate nonahydrate | Iron(III) nitrate nonahydrate | Yttrium nitrate n-hydrate | Iron(III) nitrate nonahydrate |
| Metal ion concentration 1 (wt%) | 7.7 | 0.17 | 0.23 | 10.7 | 0.17 |

(continued)

|  | Solution 1 | Solution 2 | Solution 3 | Solution 4 | Solution 5 |
|---|---|---|---|---|---|
| Metal salt 2 | Praseodymium nitrate n-hydrate | Nickel nitrate hexahydrate | Nickel nitrate hexahydrate | Praseodymium nitrate n-hydrate | Nickel nitrate hexahydrate |
| Metal ion concentration 2 (wt%) | 0.3 | 0.09 | 0.13 | 0.3 | 0.09 |
| Metal salt 3 | Neodymium nitrate hexahydrate | Zirconium oxynitrie | Zirconium oxynitrie | Neodymium nitrate hexahydrate | - |
| Metal ion concentration 3 (wt%) | 0.18 | 7 | 7 | 0.18 | - |
| Additive 1 | Propylene glycol 25wt% | Propylene glycol 25wt% | Propylene glycol 25wt% | Propylene glycol 10wt% | Propylene glycol 25wt% |
| Additive 2 | - | - |  | - | 5 |
|  |  |  |  |  |  |
|  | Solution 6 | Solution 7 | Solution 8 | Solution 9 | Solution 10 |
| Solvent | Ion exchange water | Ion exchange water | Ion exchange water | Ion exchange water | Ion exchange water |
| Metal salt 1 | Iron(III) nitrate nonahydrate | Yttrium nitrate n-hydrate | Yttrium nitrate n-hydrate | Yttrium nitrate n-hydrate | Yttrium nitrate n-hydrate |
| Metal ion concentration 1 (wt%) | 0.23 | 11.7 | 4.4 | 8.5 | 9 |
| Metal salt 2 | Nickel nitrate hexahydrate | Praseodymium nitrate n-hydrate | Praseodymium nitrate n-hydrate | - | - |
| Metal ion concentration 2 (wt%) | 0.13 | 0.3 | 0.3 | - | - |
| Metal salt 3 | - | Neodymium nitrate hexahydrate | Neodymium nitrate hexahydrate | - | - |
| Metal ion concentration 3 (wt%) | - | 0.18 | 0.18 | - | - |
| Additive 1 | Propylene glycol 25wt% | - | Propylene glycol 25wt% | Urea 34.5wt% | Urea 34.5wt% |
| Additive 2 | 8 | - |  | - | - |

(Impregnation of impregnating solution into zirconia calcined body)

The zirconia calcined body was placed on a horizontal workbench, and a jig was attached so that the impregnating solution could be held on the top surface of the zirconia calcined body. The impregnating solution (I) (solution (I)) was poured into the top surface of the zirconia calcined body in the specified amount described in Table 3 and allowed to stand until the entire amount was absorbed by the zirconia calcined body. The impregnating solution (II) (solution (II)) was poured into the top surface of the zirconia calcined body in the specified amount described in Table 3 and allowed to stand until the entire amount was absorbed by the zirconia calcined body. The impregnating solution (III) (solution (III)) was poured into the top surface of the zirconia calcined body in the specified amount described in Table 3 and allowed to stand until the entire amount was absorbed by the zirconia calcined body. The above operation was performed for all impregnating solutions. For Example 11 and Comparative Example 1, impregnation of the impregnating solution was not performed.

(Heat treatment after impregnation)

In the case that the impregnating solution (solution) contained urea, heat treatment was performed after impregnation of the impregnating solution. The zirconia calcined body after impregnation was placed in a resin bag and degassed. The zirconia calcined body placed in the resin bag and degassed was placed in a dryer, and then a heat treatment at 95 °C for 15 hours was performed to deposit a metal compound. After the heat treatment, the zirconia calcined body was taken out from the resin bag and dried (120 °C, 1 hour) to prepare a zirconia calcined body supporting a metal compound.

(Removal of residual organic matter)

The zirconia calcined body after impregnation and/or heat treatment was heat treated in an electric furnace (500 °C, 30 min) to prepare a zirconia mill blank for dental cutting and machining in which a metal compound is supported in a pore. The calcined body 5 was also heat treated (500°C for 30 minutes) in an electric furnace.

(Repeat of impregnation)

In Examples 2, 4, and Comparative Example 2, the steps from the impregnating step to the removal of residual organic matter were repeated multiple times.

(Preparation of test specimen)

By using a dental milling machine (DWX51D, manufactured by Roland Corporation), the surface to which the impregnating solution was poured during impregnation was defined as the surface B, the surface of opposite side was defined as the surface A, and the surface which was parallel to the surface A and was positioned at 1.0 mm from the surface A toward the surface B was defined as the surface C, and a test specimen having a diameter of 12 mm and a thickness of 2.0 mm was prepared so that the surface C was the top surface of the test specimen. By the same manner, the test specimens having a diameter of 12 mm and a thickness of 2.0 mm were prepared so that the top surface of the test specimen was constituted with the surface which was positioned at 0.5 mm, 1.0 mm, 1.5 mm, 4.0 mm, 4.5 mm, 5.0 mm, 5.5 mm, 8.0 mm, 8.5 mm, 9.0 mm, 9.5 mm, 12.0 mm, 12.5 mm, 13.0 mm, 13.5 mm or 14.0 mm from the surface C toward the surface B. For Comparative Example 1, impregnation was not performed and there is no distinction between the surface A and the surface B. Therefore, any face was designated as the surface A, and the test specimen was prepared in the same manner.

(Evaluation of amount of stabilizer)

A molar fraction of stabilizer in terms of oxide (stabilizer oxide: example $Y_2O_3$) contained in each test specimen was measured by using a fluorescent X-ray apparatus (manufactured by Rigaku Corporation). In Examples and comparative examples, yttrium, praseodymium and neodymium were detected as stabilizer, and the total of these stabilizer concentrations was defined as the stabilizer concentration for each specimen. Measurements were performed on the top surface and the bottom surface of the test specimen, and each measurement was defined as the stabilizer concentration at each distance from the surface C in the zirconia calcined body. For example, in the case of a test specimen in which the top surface is constituted with the surface which is positioned at 1.0 mm from the surface C toward the surface B, the stabilizer concentration at the top surface and the stabilizer concentration at the bottom surfaces can be regarded as the stabilizer concentrations at 1.0 mm and 3.0 mm from the surface C toward the surface B. The stabilizer concentrations measured in this way were defined as, in order from the surface C, point P(1), point P(2), ... point P(33), and the maximum value among them was defined as SPmax and the minimum value among them was defined as SPmin. The difference between the value of point P(1) and the value of point P(2) was defined as $\Delta$SP(1), and $\Delta$SP(2), $\Delta$SP(3), ... $\Delta$SP(32) were defined for all points P in the same manner, and the maximum value among them was defined as $\Delta$SPmax. Furthermore, the absolute value of the difference between $\Delta$SP (1) and $\Delta$SP (2) was defined as $\Delta\Delta$SP (1), and $\Delta\Delta$SP (2), $\Delta\Delta$SP (3), ... $\Delta\Delta$SP (31) were defined for all $\Delta$SP in the same manner.

(Evaluation of translucency)

Each test specimen after evaluation of amount of stabilizer was perfect sintered (firing temperature: 1550 °C, holding time: 1 hours) in a firing furnace. Then, each test specimen was adjusted to have the thickness (1.0 mm) with a surface grinder. The translucency was evaluated by measuring the contrast ratio. The contrast ratio was measured by using a spectrocolorimeter (manufactured by Konica Minolta). In the following formula, Yw is the value Y measured by placing the white plate behind the test specimen, and Yb is the value Y measured by placing the black plate behind the test specimen. The contrast ratio was calculated from the following formula. When the contrast ratio value is close to zero, the materials are seen as transparency. When the contrast ratio value is close to 1, the materials are seen as opaqueness.

Formula: The contrast ratio $= Yb / Yw$

(Evaluation of appearance)

A plate test specimen (18 mm × 18 mm × 2.0 mm) perpendicular to the surface A was cut out from a zirconia mill blank for dental cutting and machining. Each test specimen was perfect sintered (firing temperature: 1550 °C, holding time: 1 hours) in a firing furnace. Then, the test specimen was adjusted to have the thickness (0.5 mm) with a surface grinder. The appearance of the prepared test specimen was visually evaluated. The evaluations were performed by five technicians, and the results of the most frequent evaluations are shown in Table 2.

AA: It had natural gradation and translucency similar to a natural tooth, and can be used sufficiently in cases requiring high aesthetic property.
A: It had natural gradation and/or translucency similar to a natural tooth, and can be used to some extent in cases requiring high aesthetic property.
B: It had some degree of natural gradation and/or translucency similar to a natural tooth, and may be used in cases requiring high aesthetic property.
C: Natural gradation cannot be obtained and/or it did not have the translucency similar to a natural tooth and it cannot be used in cases requiring high aesthetic property.

Table 2 shows the characteristic test results of the prepared zirconia mill blank for dental cutting and machining in Examples and Comparative Examples.

[Table 2]

| | | Example 1 | Example 2 | Example 3 | Example 4 | Example 5 | Example 6 | Example 7 | Example 8 |
|---|---|---|---|---|---|---|---|---|---|
| Zirconia calcined body | | Calcined body 1 | Calcined body 2 | Calcined body 1 | Calcined body 3 | Calcined body 4 | Calcined body 6 | Calcined body 2 | Calcined body 3 |
| Type of impregnating liquid | Solution (I) | Solution 1 | Solution 4 | Solution 1 | Solution 7 | Solution 1 | Solution 1 | Solution 1 | Solution 1 |
| | Solution (II) | Solution 2 | Solution 2 | Solution 5 | Solution 5 | Solution 5 | Solution 5 | Solution 5 | Solution 5 |
| | Solution (III) | Solution 3 | Solution 3 | Solution 6 | Solution 6 | Solution 6 | Solution 6 | Solution 6 | Solution 6 |
| Amount of impregnating liquid (g) | Solution (I) | 22.4 | 22.4 | 22.4 | 22.4 | 22.4 | 22.4 | 22.4 | 22.4 |
| | Solution (II) | 12.8 | 128 | 128 | 128 | 128 | 128 | 128 | 128 |
| | Solution (III) | 28.8 | 28.8 | 28.8 | 28.8 | 28.8 | 28.8 | 28.8 | 28.8 |
| Impregnation repetition number | | 1 | 2 | 1 | 3 | 1 | 1 | 1 | 1 |
| SPmax - Spmin | | 0.90 | 2.24 | 0.69 | 2.97 | 2.07 | 1.71 | 0.68 | 0.70 |
| Spmax | | 6.35 | 6.10 | 6.34 | 6.19 | 6.36 | 6.34 | 4.95 | 3.98 |
| Contrast ratio | | 0.63 | 0.66 | 0.63 | 0.65 | 0.62 | 0.62 | 0.72 | 0.75 |
| △SPmax | | 0.096 | 0.213 | 0.085 | 0.385 | 0.370 | 0.303 | 0.075 | 0.098 |
| Number of paortions satisfing the formula of △△SP(m) | | 0 | 0 | 0 | 6 | 8 | 10 | 0 | 0 |
| Evaluation of appearance | | AA | A | AA | A | A | A | A | A |

|  |  | Example 9 | Example 10 | Example 11 | Example 12 | Comparative Example 1 | Comparative Example 2 | Comparative Example 3 |
|---|---|---|---|---|---|---|---|---|
| Zirconia calcined body |  | Calcined body 1 | Calcined body 2 | Calcined body 7 | Calcined body 3 | Calcined body 5 | Calcined body 1 | Calcined body 1 |
| Type of impregnating liquid | Solution (I) | Solution 9 | Solution 10 | - | Solution 7 | - | Solution 7 | Solution 8 |
|  | Solution (II) | - | - | - | Solution 5 | - | Solution 5 | Solution 5 |
|  | Solution (III) | - | - | - | Solution 6 | - | Solution 6 | Solution 6 |
| Amount of impregnating liquid (g) | Solution (I) | 6.1 | 6.1 | - | 11.0 | - | 22.4 | 22.4 |
|  | Solution (II) |  |  | - | 242 | - | 128 | 128 |
|  | Solution (III) | - | - | - | 28.8 | - | 28.8 | 28.8 |
| Impregnation repetition number |  | 1 | 1 | 0 | 3 |  | 4 | 1 |
| SPmax - Spmin |  | 0.75 | 0.79 | 2.84 | 2.93 | 2.44 | 3.92 | 0.29 |
| Spmax |  | 6.40 | 508 | 6.12 | 6.18 | 5.72 | 7.16 | 5.98 |
| Contrast ratio |  | 0.62 | 0.69 | 0.67 | 0.62 | 0.71 | 0.62 | 0.70 |
| △SPmax |  | 0.293 | 0.305 | 0.230 | 0.486 | 0.584 | 0.513 | 0.035 |
| Number of paortions satisfing the formula of △△SP(m) |  | 1 | 1 | 8 | 2 | 9 | 8 | 0 |
| Evaluation of appearance |  | A | B | B | B | C | C | C |

EP 4 371 967 A1

In Examples 1 to 12, the value of "SPmax-SPmin" is 0.3 or more, and the maximum value ΔSPmax of ΔSP(m) is less than 0.5. Therefore, it was confiremd that natural gradation and translucency similar to a natural tooth was exhbited and these can be applied to cases where high aesthetic property is required.

In Comparative Examples 1 and 2, because the value of the ΔSPmax is 0.5 or more, it was confirmed that natural gradation cannot be obtained, for example boundaries where translucency changes is visible, and thus it cannot be applied to cases where high aesthetic property is required.

In Comparative Example 3, since the value of "SPmax - Spmin" was less than 0.3, it was confirmed that the gradation of translucency was not confirmed, and thus it cannot be applied to cases where high aesthetic property is required.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

Industrial applicability

The present invention relates to a zirconia mill blank for dental cutting and machining that can impart translucency gradation and color gradation similar to a natural tooth to a zirconia perfect sintered body without the need for special equipment, and is a technology that can be used in the dental field.

**Claims**

1. A zirconia mill blank for dental cutting and machining comprising

   two opposing surfaces and a side surface between the two opposing surfaces, wherein
   the side surface has a rectangular shape in a side view of the zirconia mill blank for dental cutting and machining, and
   in a case in which

   one of the two opposing surfaces is defined as surface A,
   the other surface of the two opposing surfaces is defined as surface B,
   a surface which is parallel to the surface A and is located at a position of 1.0 mm to 1.5 mm from the surface A toward the surface B in a direction parallel to the side surface is defined as surface C,
   points which are set to having 0.5 mm of distance on the straight line in the direction parallel to the side surface from the surface C toward the surface B in the area from the surface C to the surface B are defined as point P(1), point P(2), to point P(n) in the order from the surface C,
   a stabilizer concentration (mol%) at each point is defined as SP(1), SP(2), to SP(n) (n ≥ ((shortest distance (mm) between the surface C and the surface B) - 2)/0.5),
   the maximum value of the stabilizer concentration (mol%) is defined as SPmax,
   the minimum value of the stabilizer concentration (mol%) is defined as SPmin, and
   the maximum value of ΔSP(m) defined by the following formula is defined as ΔSPmax,

   the zirconia mill blank for dental cutting and machining has a portion where the value of "SPmax - Spmin" which is a difference between the SPmax and the SPmin is 0.3 or more, and the ΔSPmax is less than 0.5.

   $$\Delta SP(m) = |SP(m) - SP(m+1)| \ (m=1, 2, \ldots n-1)$$

2. The zirconia mill blank for dental cutting and machining according to claim 1, wherein the ΔSPmax is less than 0.3.

3. The zirconia mill blank for dental cutting and machining according to claim 1, wherein the ΔSPmax is less than 0.10.

4. The zirconia mill blank for dental cutting and machining according to any one of claims 1 to 3, wherein the number of three consecutive points that satisfy the following formula is less than 3.

$$\Delta\Delta SP\,(m) = |\Delta SP(m) - \Delta SP(m+1)| > 0.1\ (m = 1, 2,\ldots n\text{-}1)$$

5. The zirconia mill blank for dental cutting and machining according to any one of claims 1 to 4, wherein the SPmax which is the maximum value of stabilizer concentration is 6.0 mol% or more.

6. The zirconia mill blank for dental cutting and machining according to any one of claims 1 to 5, wherein

   in a case in which a contrast ratio is measured for a sintered test specimen having a thickness of 1.0 mm which is prepared by sintering a test specimen having a thickness of 2.0 mm cut out from a region from the surface C to the surface B at 1550°C for 1 hour to prepare a sinterd body and adjusting a thickness of the sinterd body to 1.0mm,
   the lowest contrast ratio in the region is 0.70 or less.

7. The zirconia mill blank for dental cutting and machining according to any one of claims 1 to 6, wherein the zirconia mill blank for dental cutting and machining is colored.

8. The zirconia mill blank for dental cutting and machining according to any one of claims 1 to 7, wherein the zirconia mill blank for dental cutting and machining does not have stacked structure.

9. The zirconia mill blank for dental cutting and machining according to any one of claims 1 to 8, wherein a stabilizer compound and/or a colorant compound are supported in a pore.

10. The zirconia mill blank for dental cutting and machining according to any one of claims 1 to 9, wherein the point P(1) on the surface C is positioned at a intersection of the surface C and the side surface.

11. The zirconia mill blank for dental cutting and machining according to any one of claims 1 to 9, wherein the point P(1) on the sufrace C is positioned at a point closest proximity to the surface B on the surface C.

12. The zirconia mill blank for dental cutting and machining according to any one of claims 1 to 9, wherein in the entire of the surface C, the value of "SPmax - Spmin" is 0.3 or more and the ΔSPmax is less than 0.5.

$$\Delta SP(m) = |SP(m) - SP(m+1)|\ (m=1, 2, \ldots n\text{-}1)$$

13. A method for imparting a translucency gradation and a color tone gradation to a zirconia mill blank for dental cutting and machining, comprising
   a step of impregnating the zirconia mill blank for dental cutting and machining with at least two types of impregnating solutions.

EP 4 371 967 A1

Europäisches Patentamt
European Patent Office
Office européen des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/105848 A1 (3M INNOVATIVE PROPERTIES CO [US]) 3 June 2021 (2021-06-03) * examples 3-5; tables 1,2 * | 1-13 | INV. C04B41/00 C04B41/52 C04B35/486 C04B35/632 |
| X,D | WO 2021/023791 A1 (VITA ZAHNFABRIK H RAUTER GMBH & CO KG [DE]) 11 February 2021 (2021-02-11) * figure 1; tables 1,2 * | 1-7, 10-12 | C04B35/634 C04B35/638 A61C13/00 A61C13/083 A61K6/818 |
| X | EP 2 995 434 A1 (KURARAY NORITAKE DENTAL INC [JP]) 16 March 2016 (2016-03-16) * figures 2-7; examples 1-16; tables 1-38 * | 1-7, 10-12 | A61K6/822 ADD. C04B111/00 C04B111/82 |
| X | EP 2 573 060 A1 (LIAONING UPCERA CO LTD [CN]) 27 March 2013 (2013-03-27) * paragraphs [0005], [0011], [0015]; example 8 * | 1-13 | |

TECHNICAL FIELDS SEARCHED (IPC)

C04B
A61K
A61C

The present search report has been drawn up for all claims

| Place of search | Date of completion of the search | Examiner |
|---|---|---|
| Munich | 5 April 2024 | Bonneau, Sébastien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................
& : member of the same patent family, corresponding document

EPO FORM 1503 03.82 (P04C01)

18

## EP 4 371 967 A1

### ANNEX TO THE EUROPEAN SEARCH REPORT
### ON EUROPEAN PATENT APPLICATION NO.

EP 23 20 6601

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2024

| Patent document cited in search report | | Publication date | Patent family member(s) | | Publication date |
|---|---|---|---|---|---|
| WO 2021105848 | A1 | 03-06-2021 | EP | 4065038 A1 | 05-10-2022 |
| | | | US | 2022402830 A1 | 22-12-2022 |
| | | | WO | 2021105848 A1 | 03-06-2021 |
| WO 2021023791 | A1 | 11-02-2021 | EP | 4010299 A1 | 15-06-2022 |
| | | | EP | 4010300 A1 | 15-06-2022 |
| | | | JP | 2022543125 A | 07-10-2022 |
| | | | JP | 2022544088 A | 17-10-2022 |
| | | | US | 2022273403 A1 | 01-09-2022 |
| | | | US | 2022289632 A1 | 15-09-2022 |
| | | | WO | 2021023788 A1 | 11-02-2021 |
| | | | WO | 2021023791 A1 | 11-02-2021 |
| EP 2995434 | A1 | 16-03-2016 | CN | 105189067 A | 23-12-2015 |
| | | | CN | 107417274 A | 01-12-2017 |
| | | | CN | 111499379 A | 07-08-2020 |
| | | | EP | 2995434 A1 | 16-03-2016 |
| | | | JP | 6473081 B2 | 20-02-2019 |
| | | | JP | 7233395 B2 | 06-03-2023 |
| | | | JP | 2019084830 A | 06-06-2019 |
| | | | JP | 2020114798 A | 30-07-2020 |
| | | | JP | WO2014181828 A1 | 23-02-2017 |
| | | | KR | 20160007574 A | 20-01-2016 |
| | | | KR | 20170027877 A | 10-03-2017 |
| | | | KR | 20180027612 A | 14-03-2018 |
| | | | US | 2016074142 A1 | 17-03-2016 |
| | | | US | 2019151055 A1 | 23-05-2019 |
| | | | WO | 2014181828 A1 | 13-11-2014 |
| EP 2573060 | A1 | 27-03-2013 | CN | 102344285 A | 08-02-2012 |
| | | | EP | 2573060 A1 | 27-03-2013 |
| | | | ES | 2719818 T3 | 16-07-2019 |
| | | | KR | 20140002801 A | 08-01-2014 |
| | | | PT | 2573060 T | 30-04-2019 |
| | | | TR | 201904442 T4 | 22-04-2019 |
| | | | US | 2013115365 A1 | 09-05-2013 |
| | | | WO | 2013003990 A1 | 10-01-2013 |

**REFERENCES CITED IN THE DESCRIPTION**

**Patent documents cited in the description**

- WO 2021023791 A